(19) Europäisches Patentamt
European Patent Office
Office européen des brevets

(11) Publication number: **0 106 793**
**A1**

(12) # EUROPEAN PATENT APPLICATION

(21) Application number: 83730086.2

(22) Date of filing: 09.09.83

(51) Int. Cl.³: C 07 J 53/00
A 61 K 31/585, A 61 K 31/565

(30) Priority: 17.09.82 DE 3234972

(43) Date of publication of application: 25.04.84 Bulletin 84/17

(84) Designated Contracting States: AT BE CH DE FR GB IT LI LU NL SE

(71) Applicant: SCHERING AKTIENGESELLSCHAFT Berlin und Bergkamen
Müllerstrasse 170/178 Postfach 65 03 11
D-1000 Berlin 65(DE)

(72) Inventor: Nickisch, Klaus, Dr.
Alt-Lichtenrade 11
D-1000 Berlin 49(DE)

(72) Inventor: Bittler, Dieter
Bölkauer Pfad 11
D-1000 Berlin 27(DE)

(72) Inventor: Laurent, Henry, Dr.
Glambecker Weg 21
D-1000 Berlin 28(DE)

(72) Inventor: Wiechert, Rudolf, Prof. Dr.
Petzower Strasse 8a
D-1000 Berlin 39(DE)

(72) Inventor: Losert, Wolfgang, Prof. Dr.
Landshuter Strasse 22
D-1000 Berlin 30(DE)

(54) **7-Alpha-alkyl-17-alpha-pregn-4-ene-21,17-carbolactones and -21-carboxylic acid salts and procedures for manufacturing them and pharmaceutical preparations containing them.**

(57) 7α-substituted-17α-pregn-4-ene-21, 17-carbolactones and -21-carboxylic acid salts of formula I.

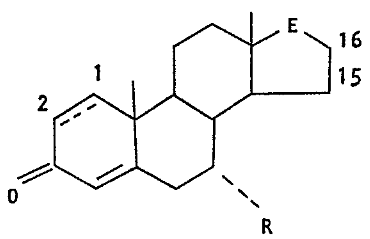

(I),

wherein
$C_1 \text{---} C_2$ is a C-C single or C=C double bond,

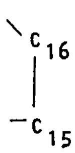

is

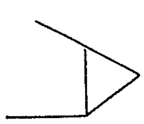

or

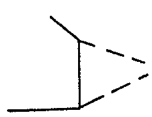

R is a saturated or unsaturated, straight-chain or branched aliphatic radical of 1-6 carbon atoms, optionally substituted by a chlorine atom or a hydroxy group, and

E is

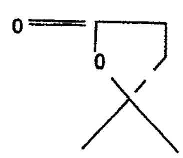

or

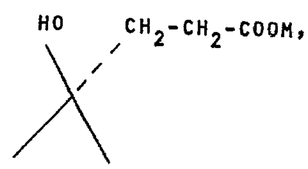

wherein M is an alkali metal, (e.g., Na, K), preferably potassium exhibit a strong antialdosterone effect and only a very slight antiandrogenic side effect.

EP 0 106 793 A1

## BACKGROUND OF THE INVENTION

The present invention relates to 7α-substituted -17α-pregn-4-ene-21,17-carbolactones and -21-carboxylic acid salts, procedures for manufacturing them and pharmaceutical preparations containing them.

In treatment with spironolactone there are frequently encountered undesirable endocrine side effects that are caused by the antiandrogenic and gestagenic activity of spironolactone. Thus, the occurrence of gynecomastia is observed in extended treatment of male patients with spironolactone (Smith, W.G., The Lancet 1962, p 886; Mann, N.M., JAMA 1963, p 778; Clark, E., JAMA 1965, p 157; Greenblatt, D.J., JAMA 1973, p 82) and impotence (Greenblatt, D.J., JAMA 1973, p 82). This can be traced to the antiandrogenic side effect of this compound (Steelman, S.L. et al, Steroids 1963, p 449; Schane, H.P., J of Clinical Endocrinology and Metabolism 1978, p 691).

The side effects on women being treated with spironolactone, such as amenorrhea and menstrual irregularities, on the other hand, are ascribed to the gestagenic side effect of spironolactone. Both side effects can be demonstrated in animal experiments as well as *in vitro* using the receptor binding test with the androgen or gestagen receptor.

## SUMMARY OF THE INVENTION:

It is an object of this invention to provide compounds that are, preferably, at least a little better

than spironolactone in antialdosterone effect, but which have strongly reduced antiandrogenic and gestagenic side effects.

Upon further study of the specification and appended claims, further objects and advantages of this invention will become apparent to those skilled in the art.

These objects have been achieved by this invention by providing 7α-substituted-17α-pregn-4-ene-21, 17-carbolactones and -21-carboxylic acid salts of formula I.

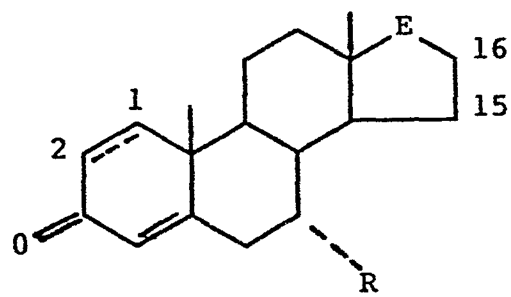

(I),

wherein

$C_1$---$C_2$ is a C-C single or C=C double bond,

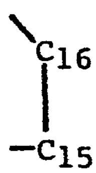 is 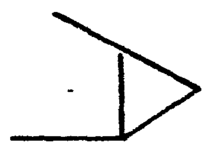 or 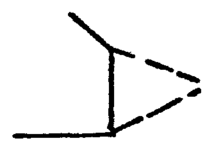

R is a saturated or unsaturated, straight-chain or branched aliphatic radical of 1-6 carbon atoms, optionally substituted by a chlorine atom or a hydroxy group, and

E is

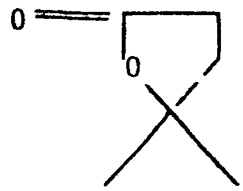

or

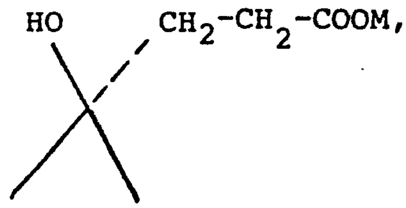

wherein M is an alkali metal, (e.g., Na, K), preferably potassium.

DETAILED DISCUSSION:

The compounds of formula I contain in the 7α -position a saturated or unsaturated straight-chain or branched aliphatic radical R of 1-6 carbon atoms optionally substituted by a chlorine atom or a hydroxyl group, for example, alkyl or alkenyl, e.g., methyl, ethyl, propyl, butyl, pentyl, hexyl, isobutyl, isobutenyl, chloropropyl, or hydroxypropyl, the propyl and butyl groups being preferred.

The new compounds of formula I have the property of neutralizing or reversing the effect of aldosterone or deoxycorticosterone on the elimination of sodium and potassium. The compounds according to this invention are thus suited for the treatment of certain forms of hypertension, edemas, primary aldosteronism, and other endocrine disturbances caused by aldosterone. They can also be used as diuretics. The new compounds have the advantage, compared with spironolactone, of having fewer side effects.

The antialdosterone effect was determined and measured in an experimental model by Hallmann (G. Hollmann et al, Tubular Effects and Renal Elimination of Spironolactones, Naunyn-Schmiedebergs Arch. Exp. Path. Pharmak. 247 (1964), p 419; P. Marx, Renal Effects of d-aldosterone

and Its Antagonist Spironolactone, Diss. Med. Fak. FU Berlin, 1966 whose disclosure is incorporated by references herein).

The androgen receptor binding test is conducted as follows:

The androgen receptor (protein) contained in the cytosol of a homogenate of rat prostates binds dihydrotestosterone (DHT) with high affinity but with low capacity. If this receptor is charged with $^3H$-DHT in the presence of the compound to be tested, the concentration and the binding affinity of the compound to be tested determine the degree to which $^3H$-DHT is displaced from the receptor. After separation of the receptor-bound DHT from the non-bound, binding can be determined in percent, and this value can be plotted against the logarithm of the molar concentration of the test substance. It is then determined what concentration of the test substance is required to displace the reference substance completely from the receptor. The competition factor (CF) as a measure of the binding strength is defined as the ratio of the concentration of the test substance to the concentration of the reference substance, so that a high CF value indicates low binding strength, whereas a low CF value indicates high affinity.

The antiandrogen effect can be determined with compounds which in themselves have no androgen effect but which through their high binding affinity fully or partially displace the body's own androgen from the receptor, as is observed to a certain degree with spironolactone. Thus a high competition factor is desirable in the androgen and gestagen receptor test.

The gestagen receptor test is conducted in the same manner using cytosol from rat uterus homogenate.

The following table summarizes the relative values of the degree of antialdosterone effect

(spironolactone = 1) and the competition factors in the androgen receptor test ($K_A$) and in the gestagen receptor test ($K_G$) of spironolactone and the compounds according to the invention as exemplified by:

I 7α-butyl-15β,16β-methylene-3-oxo-17α-pregn-4-ene-21,17-carbolactone, and

II 15β,16β-methylene-3-oxo-7α-propyl-17α-pregna-1,4-diene-21,17-carbolactone.

TABLE

| Compound | Relative antialdosterone effect | Competition factor $K_A$ | $K_G$ |
|---|---|---|---|
| Spironolactone | 1 | 8.9 | 21 |
| I | ~ 0.6 | 88 | 23 |
| II | > 1 | 112 | 55 |

The table shows that the compounds according to this invention are about as effective as spironolactone in their principal effect as aldosterone antagonists, but surprisingly exhibit as side effects a strongly reduced binding with the androgen or gestagen receptor.

7α-substituted-17α-pregn-4-ene-21,17-carbolactones containing no methyl ring in the 15,16 position are already known from European patent 0018245. In comparison with these non-methylenated compounds, the new compounds according to this invention, which contain a methyl ring in the 15,16 position, exhibit a lower antiandrogenic side effect along with about the same antialdosterone effect.

The compounds according to this invention can be formulated according to known galenic methods as medicines for oral or parenteral administration to mammals, including humans. They can be dosed and administered in the same or analogous manner as the commerical product spironolactone. Typically, dosages are 25-500 mg/day preferably - 50-400 mg/day.

Conventional excipients are pharmaceutically acceptable organic or inorganic carrier substances suitable for parenteral, enteral or topical application which do not deleteriously react with the active compounds. Suitable pharmaceutically acceptable carriers include but are not limited to water, salt solutions, alcohols, gum arabic, vegetable oils, polyethylene glycols, gelatine, lactose, amylose, magnesium stearate, talc, silicic acid, viscous paraffin, perfume oil, fatty acid monoglycerides and diglycerides, pentaerythritol fatty acid esters, hydroxy-methylcellulose, polyvinyl pyrrolidone, etc. The pharmaceutical preparations can be sterilized and if desired mixed with auxiliary agents, e.g., lubricants, preservatives, stabilizers, wetting agents, emulsifiers, salts for influencing osmotic pressure, buffers, coloring, flavoring and/or aromatic substances and the like which do not deleteriously react with the active compounds.

For parenteral application, particularly suitable are injectable sterile solutions, preferably oily or aqueous solutions, as well as suspensions, emulsions, or implants, including suppositories. Ampoules are convenient unit dosages.

For enteral application, particularly suitable are tablets, dragees, suppositories or capsules having talc and/or a carbohydrate carrier or binder or the like, the carrier preferably being lactose and/or corn starch and/or potato starch. A syrup, elixir or the like can be

used wherein a sweetened vehicle is employed. Sustained release compositions can be formulated including those wherein the active compound is protected with differentially degradable coatings, e.g., by microencapsulation, multiple coatings, etc.

Dosages for a given host for a given indication can be determined, e.g., by customary comparison of the activities of the subject compound and of a known agent by means of an appropriate, conventional pharmacological protocol.

The compounds according to this invention can be produced by reacting a compound of formula II

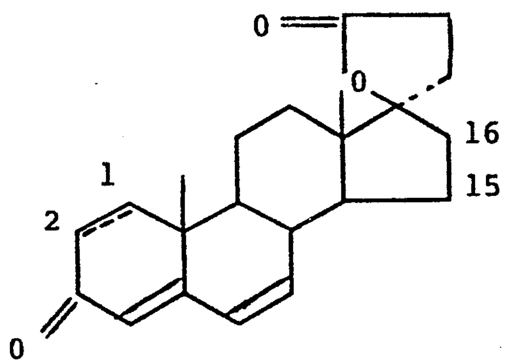

(II),

wherein

$C_1$---$C_2$ and 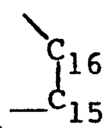 are the same as in formula I, in conventional manner with a Grignard compound of formula III

RMgHal (III)

wherein

R is as defined in formula I and Hal is a chlorine, bromine or iodine atom, in the presence of a copper salt or a compound of formula IV

$(R)_2Cu\ Li$ (IV)

R is as defined in formula I, optionally, conventionally introducing a double bond and/or optionally, conventionally splitting the lactone ring through the effect of an alkali metal hydroxide.

The reaction with the Grignard compound is conventionally carried out in the presence of a copper salt or an organometallic compound $(R)_2$ CuLi. Copper chloride, copper bromide, and copper iodide are especially suitable as copper salts.

Any solvent can be used in the reaction that is inert with respect to the reactants. Especially good are aprotic solvents miscible in water, such as tetrahydrofuran, dioxane, acetonitrile, ethylene glycol dimethyl ether, diethylene glycol dimethyl ether, and triethylene glycol dimethyl ether. The reaction is best carried out at low temperatures, preferably -30 to +20°C. To complete the process, the reaction mixture is taken up in acids. Strong acids like hydrochloric acid and sulfuric acid are suitable.

The optional subsequent introduction of the $\Delta^1$ double bond takes place according to known methods, with the use of either chemical or microbiological means. Suitable chemical dehydration agents include, for example, selenium dioxide, 2,3-dichloro-5,6-dicyanobenzoquinone, chloranil, thallium triacetate, or lead tetraacetate. Suitable microorganisms for the 1,2-dehydration include, for example, Schizomycetes, especially those of the genera Arthrobacter, e.g., A. simplex ATCC 6946; Bacillus, e.g., B. lentus ATCC 13805 and B. sphaericus ATCC 7055; Pseudomonas, e.g., P. aeruginosa IFO 3505; Flavobacterium, e.g., F. flavescens IFO 3058; Lactobacillus, e.g., L. brevis IFO 3345; and Nocardia, e.g., N. opaca ATCC 4276. Details are fully conventional.

The 1,2 dehydration is preferably carried out chemically. In this regard, the 1,2 dihydrosteroid is heated for a prolonged period in a suitable solvent with

the dehydration agent. Suitable solvents include, for example, dioxane, tert-butyl alcohol, tetrahydrofuran, toluene, benzene, or mixtures of these. The reaction is completed after several hours. It is recommended that the reaction be conducted by using thin-layer chromatography. The reaction mixture is exhausted when the initial material has been converted.

The optional subsequent splitting of the lactone ring likewise takes place according to known methods. Through the action of an alkali metal hydroxide, preferably potassium hydroxide, during heating, the alkali metal salt of 3-substituted propionic acid is formed.

The starting materials of formula II used in the process according to this invention are described in the German laid-open application 26 52 761 whose disclosure is incorporated by references herein. All starting materials can be prepared using fully conventional methods from known starting materials.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever. In the following example(s), all temperatures are set forth uncorrected in degrees Celsius; unless otherwise indicated, all parts and percentages are by weight.

Example 1:

150 mg of copper chloride at -5°C is added to a Grignard solution consisting of 486 mg of magnesium and 2.68 ml of bromobutane in 40 ml of tetrahydrofuran. To this is added by drops 1.76 g of 15β, 16β-methylene-3-oxo-17α-pregna-4,6-diene-21,17-carbolactone (DE OS 26 52 761) in 20 ml of tetrahydrofuran, and the mixture is stirred

for 2.5 hours at a temperature of 0°C. It is then poured into 400 ml of a 1 normal solution of hydrochloric acid, extracted with dichloromethane, washed with a neutral solution of sodium bicarbonate and water, dried over magnesium sulfate, and concentrated in a vacuum. The raw product is purified through column chromatography on silica gel. 525 mg of 7α-butyl-15β, 16β-methylene-3-oxo-17α-pregn-4-ene-21,17-carbolactone is obtained.

IR ($CHCl_3$): 1760, 1660, 1620 nm
UV (MeOH): $\varepsilon_{241}$ = 16 000
[$\alpha_D$] = +45.9° (C = 0.5 in chloroform).

Example 2:

500 mg of dichlorodicyanoquinone is added to a solution of 500 mg of 7α-butyl-15β, 16β-methylene-3-oxo-17α-pregn-4-ene-21,17-carbolactone in 20 ml of benzene and heated for 24 hours at the boiling point. After cooling, it is diluted with ether, washed with a solution of sodium bicarbonate and water, dried over magnesium sulfate, and evaporated in a vacuum. The residue is purified through column chromatography on silica gel. 305 mg of 7α-butyl-15β, 16β-methylene-3-oxo-17α-pregna-1,4-diene-21,17-carbolactone is obtained.

IR (KBr): 1760, 1660, 1620, 1600 $cm^{-1}$
UV (MeOH): $\varepsilon_{244}$ = 15 500.

Example 3:

Analogous to Example 1, 1.76 g of 15β, 16β-methylene-3-oxo-17α-pregna-4,6-diene-21,17-carbolactone with 2.27 ml of bromopropane and 486 mg of magnesium produces 476 mg of 15β, 16β-methylene-3-oxo-7α-propyl-17α-pregn-4-ene-21,17-carbolactone.

IR ($CHCl_3$): 1760, 1660, 1620 $cm^{-1}$
UV (MeOH):$\varepsilon_{241}$ = 16 750
$F_p$.: 220-222°C
[$\alpha_D$] = +68° ($CHCl_3$ 0.5%).

Example 4:

Analogous to Example 2, 420 mg of 15β, 16β-methylene-3-oxo-7α-propyl-17α-pregn-4-ene-21,17-carbolactone and 420 mg of dichlorodicyanoquinone produce 265 mg of 15β, 16β-methylene-3-oxo-7α-propyl-17α-pregna-1,4-diene-21,17-carbolactone.

IR (KBr): 1760, 1660, 1620, 1600 $cm^{-1}$
UV (MeOH):$\varepsilon_{244}$ = 16 000
[$\alpha_D$] = -1.4° ($CHCl_3$ 0.5%).

Example 5:

Analogous to Example 1, 1.76 g of 15β, 16β-methylene-3-oxo-17α-pregna-4,6-diene-21,17-carbolactone (DE OS 265 27 61) with 2.68 ml of bromobutane and 486 mg of magnesium produce 595 mg of 7α-butyl-15β, 16β-methylene-3-oxo-17α-pregn-4-ene-21,17-carbolactone.

IR (KBr): 1760, 1660, 1620 $cm^{-1}$
UV (MeOH):$\varepsilon_{240}$ = 15 800.

Example 6:

Analogous to Example 2, 380 mg of 7α-butyl-15β,16β-methylene-3-oxo-17α-pregn-4-ene-21,17-carbolactone and 380 mg of dichlorodicyanoquinone produce 210 mg of 7α-butyl-15β,16β-methylene-3-oxo-17α-pregna-1,4-diene-21,17-carbolactone.

IR (KBr): 1760, 1660, 1620 $cm^{-1}$
UV (MeOH):$\varepsilon_{243}$ = 15 400

Example 7:

Analogous to Example 1, 1.76 g of 15α,16α-methylene-3-oxo-17α-pregna-4,6-diene-21,17-carbolactone with 2.26 ml of bromopropane and 486 mg of magnesium produce 615 mg of 15α,16α-methylene-3-oxo-7α-propyl-17α-pregn-4-ene-21,17-carbolactone.

IR (KBr): 1760, 1660, 1620 $cm^{-1}$
UV (MeOH): $\varepsilon_{241}$ = 16 200

Example 8:

Analogous to Example 2, 425 mg of 15α,16α-methylene-3-oxo-7α-propyl-17α-pregn-4-ene-21,17-carbolactone and 425 mg of dichlorodicyanoquinone produce 225 mg of 15α,16α-methylene-3-oxo-7α-propyl-17α-pregna-1,4-diene-21,17-carbolactone.

IR (KBr): 1760, 1660, 1620, 1600 $cm^{-1}$
UV (MeOH): $\varepsilon_{244}$ = 16 500

Example 9:

1 ml of a 1 normal methanolic potassium hydroxide solution is added to a solution of 404 mg of 7α-butyl-15β, 16β-methylene-3-oxo-17α-pregn-4-ene-21,17-carbolactone in 5 ml of isopropanol and then boiled 30 minutes under reflux. After cooling it is poured into 50 ml of ice-cold ether and the precipitate is filtered and washed again with ether. After drying in a vacuum, 305 mg of 7α-butyl-17β-hydroxy-15β, 16β-methylene-3-oxo-17α-pregn-4-ene-21-carboxylic acid-potassium salt are obtained.

Example 10:

Analogous to Example 9, 402 mg of 7α-butyl-15β, 16β-methylene-3-oxo-17α-pregna-1,4-diene-21,17-carbolactone produce 325 mg of 7α-butyl-17β-hydroxy-15β,16β-methylene-3-oxo-17α-pregna-1,4-diene-21-carboxylic acid-potassium salt.

Example 11:

Analogous to Example 9, 392 mg of 15α,16α-methylene-7α-propyl-3-oxo-17α-pregn-4-ene-21,17-carbolactone produce 294 mg of 17β-hydroxy-15α,16α-methylene-3-oxo-7α-propyl-17α-pregn-4-ene-21-carboxylic acid-potassium salt.

Example 12:

Analgous to Example 9, 390 mg of 15β,16β-methylene-3-oxo-7α-propyl-17α-pregna-1,4-diene-21,17-carbolactone produce 275 mg of 17β-hydroxy-15β,16β-methylene-3-oxo-7α-propyl-17α-pregna-1,4-diene-21-carboxylic acid-potassium salt.

Example 13:

Analogous to Example 9, 404 mg of 7α-butyl-15α,16α-methylene-3-oxo-17α-pregn-4-ene-21,17-carbolactone produce 285 mg of 7α-butyl-17β-hydroxy-15α,16α-methylene-3-oxo-17α-pregn-4-ene-21-carboxylic acid-potassium salt.

Example 14:

Analogous to Example 9, 402 mg of 7α-butyl-15α,16α-methylene-3-oxo-17α-pregn-1,4-diene-21,17-carbolactone produce 332 mg of 7α-butyl-17β-hydroxy-15α,16α-methylene-3-oxo-17α-pregna-1,4-diene-21-carboxylic acid-potassium salt.

Example 15:

Analogous to Example 9, 392 mg of 15α,16α-methylene-3-oxo-7α-propyl-17α-pregn-4-ene-21,17-carbolactone produce 335 mg of 17β-hydroxy-15α,16α-methylene-3-oxo-7α-propyl-17α-pregn-4-ene-21-carboxylic acid-potassium salt.

Example 16:

Analogous to Example 9, 390 mg of 15α,16α-methylene-3-oxo-7α-propyl-17α-pregna-1,4-diene-21,17-carbolactone produce 340 mg of 17β-hydroxy-15α,16α-methylene-3-oxo-7α-propyl-17α-pregna-1,4-diene-21-carboxylic acid-potassium salt.

The preceding examples can be repeated with similar success by substituting the generically or specifically described reactants and/or operating conditions of this invention for those used in the preceding examples.

From the foregoing description, one skilled in the art can easily ascertain the essential characteristics of this invention and without departing from the spirit and scope thereof, can make various changes and modifications of the invention to adapt it to various usages and conditions.

WHAT IS CLAIMED IS:

1. A 7α-substituted-17α -pregn-4-ene-21,17-carbolactone or -21-carboxylic acid salt of the formula

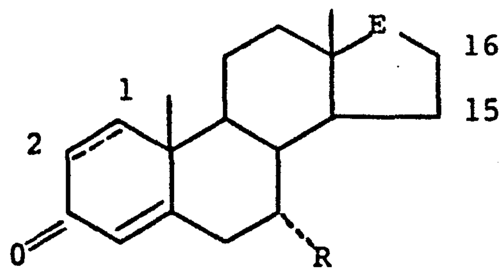

wherein

$C_1$---$C_2$ is a C-C-single- or C=C-double bond,

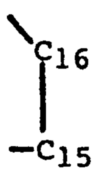 is 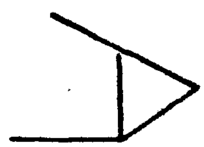 or 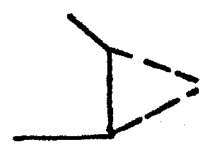

R is $C_{1-6}$-alkyl or $C_{2-6}$-alkylene, each optionally substituted by a chlorine atom or a hydroxy group and

E is 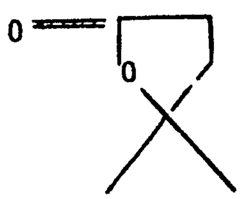 or 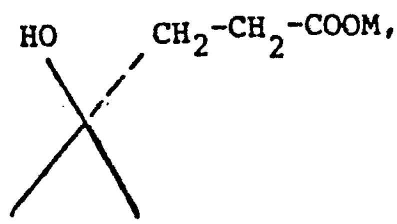

wherein M is an alkali metal atom.

2. A compound of Cl 1 wherein E is

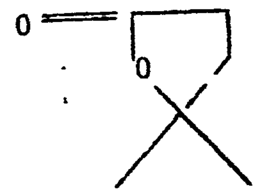

3. A compound of Cl 1 wherein E is

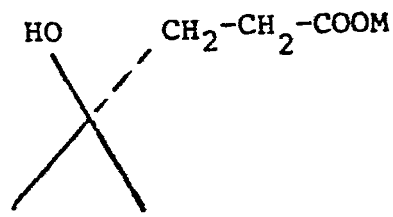

4. 7α-butyl-15β,16β -methylene-3-oxo-17α-pregn-4-ene-21,17-carbolactone, a compound of claim 1.

5. 7α-butyl-15β,16β -methylene-3-oxo-17α-pregna-1,4-diene-21,17-carbolacotne, a compound of claim 1.

6. 15β,16β -methylene-3-oxo-7α-propyl-17α -pregn-4-ene-21,17-carbolactone, a compound of claim 1.

7. 15β,16β -methylene-3-oxo-7α-propyl-17α-pregna-1,4-diene-21,17-carbolactone, a compound of claim 1.

8. 7α-butyl-15α,16α-methylene-3-oxo-17α-pregn-4-ene-21,17-carbolactone, a compound of claim 1.

9. 7α-butyl-15α,16α-methylene-3-oxo-17α-pregna-1,4-diene-21,17-carbolactone, a compound of claim 1.

10. 15α,16α-methylene-3-oxo-7α-propyl-17α-pregn-4-ene-21,17-carbolactone, a compound of claim 1.

11. 15α,16α-methylene-3-oxo-7α-propyl-17α-pregna-1,4-diene-21,17-carbolactone, a compound of claim 1.

12. 7α-butyl-17β -hydroxy-15β,16β -methylene-3-oxo-17α-pregn-4-ene-21-carboxylic acid - potassium salt, a compound of claim 1.

13. 7α-butyl-17β -hydroxy-15β,16β-methylene-3-oxo-17α-pregna-1,4-diene-21-carboxylic acid - potassium salt, a compound of claim 1.

14. 17β-hydroxy-15β,16β -methylene-3-oxo-7α-propyl-pregn-4-ene-21-carboxylic acid - potassium salt, a compound of claim 1.

15. 17β-hydroxy-15β,16β-methylene-3-oxo-7α--propyl-17α-pregna-1,4-diene-21-carboxylic acid - potassium salt, a compound of claim 1.

16. 7α-butyl-17β-hydroxy-15α,16α-methylene-3-oxo-17α-pregn-4-ene-21-carboxylic acid - potassium salt, a compound of claim 1.

17. 7α-butyl-17β-hydroxy-15α,16α-methylene-3-oxo-17α-pregna-1,4-diene-21-carboxylic acid - potassium salt, a compound of claim 1.

18. 17β-hydroxy-15α,16α-methylene-3-oxo-7α-propyl-17α-pregn-4-ene-21-carboxylic acid - potassium salt, a compound of claim 1.

19. 17β-hydroxy-15α,16α-methylene-3-oxo-7-α-propyl-17α-pregna-1,4-diene-21-carboxylic acid - potassium salt, a compound of claim 1.

20. A pharmaceutical composition comprising an antialdosterone effective amount of a compound of claim 1 and a pharmaceutically acceptable carrier.

21. A method of achieving an antialdosterone effect in a patient in need of the same comprising administering to the patient an effective amount of a compound of claim 1 to the patient.

22. A procedure for manufacturing 7α-substituted-17α-pregn-4-ene-21,17-carbolactones or -21-carboxylic acid salts of the formula I

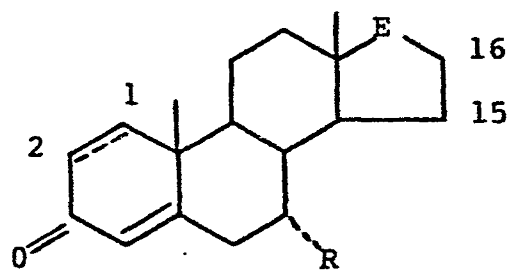

wherein

$C_1$---$C_2$ is a C-C-single- or C=C-double bond,

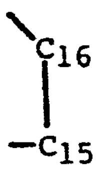 is 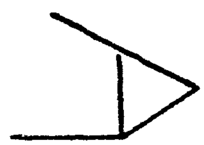 or 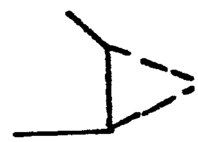

R is $C_{1-6}$-alkyl or $C_{2-6}$-alkylene, each optionally substituted by a chlorine atom or a hydroxy group and

E is 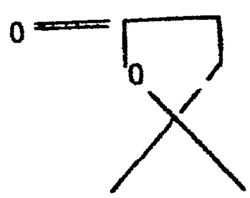 or 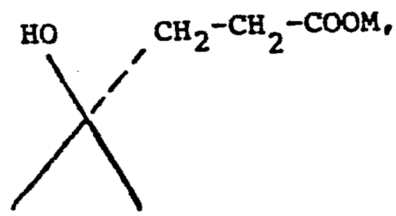

wherein M is an alkali metal atom, comprising,

reacting a compound of formula II

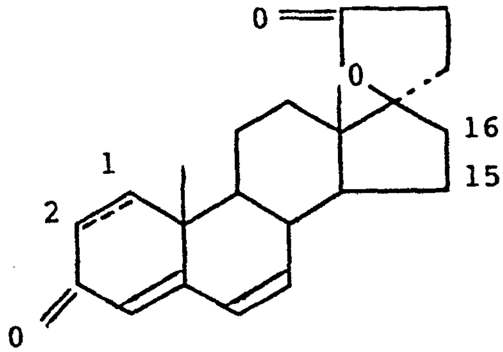

(II),

wherein $C_1 \overset{---}{} C_2$ and 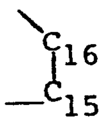 are the same as in formula I, in conventional manner with a Grignard compound of formula III

RMgHal (III)

wherein
R is as defined in formula I and Hal is a chlorine, bromine or iodine atom, in the presence of a copper salt or a compound of formula IV

$(R)_2Cu\ Li$ (IV)

wherein
R is a defined in formula I, optionally, conventionally introducing a double bond and/or optionally, conventionally splitting the lactone ring through the effect of an alkali metal hydroxide.

0106793

European Patent Office

# EUROPEAN SEARCH REPORT

Application number

EP 83 73 0086

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | EP-A-0 018 245 (ROUSSEL UCLAF)<br>* Claims 1-11 *<br>--- | 1-3,20-22 | C 07 J 53/00<br>A 61 K 31/585<br>A 61 K 31/565 |
| Y | FR-A-2 370 755 (SCHERING AG)<br>* Claims *<br>--- | 1-3,20-22 | |
| Y | FR-A-2 255 914 (SEARLE)<br>* Claims 1, 10-12 *<br>----- | 1,2,20-22 | |
| | | | TECHNICAL FIELDS SEARCHED (Int. Cl. 3) |
| | | | C 07 J 53/00 |

The present search report has been drawn up for all claims

| Place of search | Date of completion of the search | Examiner |
|---|---|---|
| THE HAGUE | 19-01-1984 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document